# FASCICULE DE BREVET EUROPEEN

(11) **EP 2 640 365 B1**
(45) Date de publication et mention de la délivrance du brevet: **10.05.2017**
(21) Numéro de dépôt: 11796756.2
(22) Date de dépôt: 18.11.2011
(51) Int. Cl.: A61K 9/28, A61K 9/50, A61K 31/16

(54) **COMPOSITION PHARMACEUTIQUE COMPRENANT DU SEL PRECURSEUR DU CYCLE DE KREBS, EN PARTICULIER DU SEL DE CITRATE, ET SON UTILISATION COMME MEDICAMENT**
PHARMAZEUTISCHE ZUSAMMENSETZUNG MIT KREBSZYKLUSVORLÄUFERSALZ, INSBESONDERE ZITRATSALZ, UND IHRE VERWENDUNG ALS ARZNEIMITTEL
PHARMACEUTICAL COMPOSITION COMPRISING KREBS CYCLE PRECURSOR SALT, IN PARTICULAR CITRATE SALT, AND USE THEREOF AS A MEDICAMENT

(30) Priorité: 18.11.2010 FR 1059468
(43) Date de publication de la demande: 25.09.2013
(73) Titulaire: Advicenne, 30000 Nîmes (FR)
(72) Inventeur: ROUSSEL-MAUPETIT, Caroline, F-38330 Saint-Ismier (FR); GRANIER, Luc-André, F-30490 Montfrin (FR); GUITTET, Catherine, F-13200 Arles (FR)
(74) Mandataire: Santarelli
(86) Numéro de dépôt international: PCT/FR2011/052691
(87) Numéro de publication internationale: WO 2012/066254

(56) Documents cités:
- EP-A2- 0 429 157
- WO-A1-97/02017
- DE-A1- 2 252 665
- US-A1- 2009 028 935
- BIYANI C S ET AL: "Cystinuria-Diagnosis and Management", EAU - EBU UPDATE SERIES, ELSEVIER, AMSTERDAM, NL, vol. 4, no. 5, 1 octobre 2006 (2006-10-01) , pages 175-183, XP024992348, ISSN: 1871-2592, DOI: DOI:10.1016/J.EEUS.2006.06.001 [extrait le 2006-10-01]
- "Urocit -K HIGHLIGHTS OF PRESCRIBING INFORMATION", fda WEBSITE, 31 décembre 2009 (2009-12-31), pages 1-4, XP055002516, Extrait de l'Internet: URL:http://www.accessdata.fda.gov/drugsatf da_docs/label/2010/019071s012lbl.pdf [extrait le 2011-07-11]

## Description

La présente demande concerne une composition pharmaceutique comprenant au moins un sel précurseur du cycle de Krebs, le citrate de potassium utilisée en particulier pour le traitement et/ou la prévention des lithiases urinaires se manifestant à un pH physiologique et/ou lors d'acidose urinaire et/ou lors d'hypocitraturie et/ou lors d'hypercalciurie et/ou lors d'hyperoxalurie.

Le pH physiologique urinaire est typiquement de l'ordre d'environ 5 à 5,5.

L'acidose urinaire et/ou l'hypocitraturie et/ou l'hypercalciurie et/ou l'hyperoxalurie sont généralement des indications de lithiase urinaire.

La lithiase urinaire est une maladie consistant en la formation de calculs dans la voie urinaire. Un calcul urinaire est constitué principalement de substances cristallines. La cristallisation dépend étroitement de la saturation des urines en composés cristallisables comme le calcium, l'oxalate, le phosphore, le magnésium, le bicarbonate, l'acide urique, l'urate, le sodium ou la cystine. Ces divers composés éliminés dans les urines interviennent donc directement par leur concentration et leur tendance à cristalliser dans la formation des calculs. Mais cette tendance est aussi influencée par diverses substances inhibitrices ou inductrices de cristallisation. Ainsi les sels précurseurs du cycle de Krebs, et en particulier l'ion citrate, ont une action inhibitrice sur la formation de certains calculs en limitant voire empêchant la croissance des cristaux, leur agrégation et leur nucléation *in vivo* et *in vitro.*

Un des traitements de la lithiase urinaire est l'alcalinisation des urines. Des compositions alcalines comprenant des sels alcalins sont généralement indiquées en traitement de l'acidose urinaire et/ou de l'hypocitraturie et/ou de l'hypercalciurie et/ou de l'hyperoxalurie, mais aussi de certaines tubulopathies dont la cystinurie, ainsi que certaines maladies métaboliques héréditaires.

Parmi les sels alcalins utilisés, on peut mentionner le citrate de potassium.

Un des avantages du citrate de potassium est qu'il est excrété par les reins. Le citrate urinaire résulte de cette excrétion tubulaire rénale, ainsi que, éventuellement, de l'élimination urinaire directe d'un excès d'apport exogène, entraînant une alcalinisation des urines. Un autre intérêt du citrate est qu'il se complexe avec le calcium dans les urines. Ainsi, c'est l'un des ions les plus utilisés pour prévenir la formation de calculs de phosphate de calcium, d'oxalate de calcium, d'acide urique et de cystine, grâce à sa capacité à alcaliniser les urines et à éliminer la sursaturation des urines en ion oxalo calcique.

Des préparations de citrate de potassium (principalement des préparations magistrales ou à libération immédiate) sont couramment utilisées pour la prévention des lithiases urinaires se manifestant à des pH physiologiques et/ou lors d'acidose urinaire et/ou lors d'hypocitraturie et/ou lors d'hypercalciurie et/ou lors d'hyperoxalurie. Il est fortement conseillé aux patients présentant de tels calculs de continuer le traitement alcalin à vie.

L'Urocit-K est une composition pharmaceutique commerciale à libération prolongée comportant du citrate de potassium et une micro-cire, et se présente sous la forme de comprimés. L'effet prolongé annoncé de l'Urocit-K a été observé *in vitro.* L'Urocit-K présente une faible tolérance gastrique. En outre, cette formulation n'est pas adaptée aux enfants du fait de la grosseur des comprimés.

Cependant, selon Harvey (J. Clin. Pharmacol. 1989 ;29 :338-341), l'Urocit-K présente un profil de dissolution *in vivo* proche de celui observé avec un profil de dissolution immédiat. En effet, Harvey a montré que des préparations liquides (à libération immédiate) et l'Urocit-K, préparation à libération prolongée, ont le même effet physiologique sur l'alcalinisation des urines. Par suite, l'Urocit-K se comporte *in vivo* comme une composition pharmaceutique à libération immédiate.

En outre, un inconvénient à l'ingestion de citrate de potassium est sa faible tolérance gastrique, qui limite les quantités pouvant être absorbées en une seule prise. Ainsi, les formulations liquides à action immédiate sont à prendre pendant les repas en fractionnant les prises tout au long de la journée. De plus, les sels de citrate ont un goût particulièrement déplaisant et peu apprécié des patients et tout particulièrement des enfants.

Des études (Hess B. Néphrolithiase. Forum Med Suisse. 2001 ;45 :1119-1127) ont montré que les traitements de patients à l'aide de sels de citrate en préparation à libération immédiate ont dû être interrompus prématurément pour 36% et 48% des patients traités par des citrates en raison du mauvais goût des médicaments et/ou d'effets secondaires principalement gastro-intestinaux (ballonnements, renvois, diarrhées), le plus souvent durant les six premiers mois. En comparaison, les traitements n'ont été interrompus que dans 12 et 31% des cas sous placebo.

Ainsi, les médicaments existants comportant en tant que principe actif du sel de citrate posent des problèmes de prise et/ou d'effets secondaires, qui limitent fortement leur utilisation. Le besoin de médicament adapté aux lithiases urinaires se manifestant à des pH physiologiques et/ou lors d'acidose urinaire et/ou lors d'hypocitraturie et/ou lors d'hypercalciurie et/ou lors d'hyperoxalurie, est persistant à ce jour.

La composition selon l'invention répond à cette problématique, et en particulier elle permet de conserver les avantages du citrate de potassium tout en palliant les inconvénients cités ci-dessus.

La composition selon l'invention est une composition pharmaceutique solide à usage oral sous forme d'au moins un comprimé, le comprimé étant constitué d'un coeur comprenant du citrate de potassium en tant que principe actif, de préférence en tant que seul principe actif, et d'un enrobage comprenant au moins un agent d'enrobage, ladite composition comprenant de 40% à 80%, de préférence de 50 à 70%, en poids de citrate de potassium sur la base du poids total de la composition, ladite composition étant apte à libérer le citrate de potassium *in vitro*, aussi bien dans un milieu de dissolution d'eau purifiée à pH 7 que dans un milieu de dissolution de solution tamponnée à pH 1,3, avec un appareil de dissolution de type 2, conforme à la Pharmacopée Européenne 2.9.3 « *Essai de dissolution des formes solides* », à un taux de 2 à 15% en 15 minutes, de 15 à 25% en 30 minutes, et de 30 à 50% en une heure. Le comprimé consiste en un coeur et un enrobage.

Typiquement et de préférence, la dissolution *in vitro* de la composition selon l'invention dans un milieu de dissolution donné, selon les conditions décrites plus haut, se produit à pH indépendant. Cela veut dire que, quelque soit le pH du milieu de dissolution dans une plage entre 1,3 et 7, la dissolution se produit de façon similaire. La demanderesse a choisi ici deux milieux de dissolution différents, chacun caractérisé par son pH, à savoir pH 1,3 et pH 7, pour définir ce profil de façon caractéristique, avantageusement selon un test facilement reproductible *in vitro.*

Ainsi, le comprimé selon l'invention est enrobé. Selon la définition de la Pharmacopée Européenne (Ph. Eur.), un comprimé enrobé est un comprimé recouvert d'une ou plusieurs couches de mélange de substances diverses telles que résine naturelles ou synthétiques, gommes, gélatine, charges insolubles inactives, sucres, substances plastifiantes, polyols, cires, colorants autorisés par l'Autorité compétente et, parfois, aromatisants et substances actives. Toutefois, selon l'invention, il est exclu que l'enrobage comprenne du citrate de potassium.

Quand l'enrobage est constitué d'un film polymère très mince, le comprimé est dit pelliculé (cf. Ph. Eur.).

Avantageusement, l'enrobage permet à la fois de masquer le goût et de gérer la cinétique de libération du citrate de potassium.

Le « coeur de comprimé » est, selon l'invention, toute la partie du comprimé qui n'est pas l'enrobage.

Par « composition pharmaceutique », on entend selon l'invention une composition dont les composants sont acceptables d'un point de vue pharmaceutique. En particulier, la composition est constituée de composants appropriés et acceptables pour une administration pharmaceutique orale.

Par « composant choisi parmi les éléments », on entend que le composant est un des éléments ou bien un mélange de ces éléments.

La composition pharmaceutique selon l'invention permet très avantageusement une libération continue *in vivo* de façon contrôlée, sur une durée généralement de quatre heures au maximum après la prise, i.e. après une prise unique. Par libération continue, on entend selon l'invention une libération qui s'effectue constamment *in vivo,* depuis la prise de la composition jusqu'à une durée d'environ quatre heures au maximum. Une telle libération est qualifiée de « prolongée » car elle atteint ou dépasse une durée d'une heure.

Cette libération contrôlée observée *in vitro* reflète une libération contrôlée dans l'organisme, qui peut être vérifiée par mesure du pH urinaire des sujets traités par cette composition, usuellement à intervalles réguliers, par exemple toutes les deux heures.

De préférence, la composition pharmaceutique selon l'invention est telle qu'elle libère *in vivo* pratiquement tout le citrate de potassium (c'est-à-dire au moins 95% dudit sel) sur une durée maximale d'environ quatre heures après une prise unique de la composition.

Sans vouloir être liée par une quelconque hypothèse, la demanderesse pense que le mécanisme d'action est tel que, lorsque la composition est administrée par voie orale à un sujet et que le précurseur du cycle de Krebs est le citrate de potassium le citrate est libéré de façon progressive et contrôlée, étant ainsi absorbé dans le duodénum et le jéjunum. De plus, du fait de cette libération que l'on peut qualifier de « lente », la possibilité d'alcalose sanguine est évitée.

Ainsi, cette cinétique de libération permet avantageusement d'optimiser la quantité de citrate de potassium administrée et par la même la quantité de citrate de potassium absorbée par l'organisme et agissant en tant qu'alcalinisant.

Par conséquent, la composition selon l'invention est particulièrement bien adaptée pour alcaliniser les urines et pour le traitement de l'acidose survenant lors de certaines maladies urinaires, et elle permet une action anti-acidose plus efficace que les formulations de l'art antérieur.

En outre, la tolérance gastrique est avantageusement améliorée par rapport aux formulations de l'art antérieur, principalement grâce à une libération progressive du principe actif pendant une durée maximum typiquement d'environ 120 à environ 240 minutes, ce qui permet d'éviter un effet de matraquage de l'organisme du patient trop important et susceptible de créer une alcalose.

Le sel de citrate est choisi parmi le citrate de potassium, le citrate de sodium et le citrate de magnésium, comme étant le citrate de potassium.

Selon l'invention, la composition selon l'invention est apte à libérer (ou dissoudre) le citrate de potassium *in vitro* dans un milieu de dissolution d'eau purifiée à pH 7 réalisé avec un appareil de dissolution de type 2, conforme à la Pharmacopée Européenne (Ph. Eur.) 2.9.3 « *Essai de dissolution des formes solides* », à un taux de 2 à 15% en 15 minutes, de 15 à 25% en 30 minutes, et de 30 à 50% en une heure.

Ce pH de 7 est une mesure aisée à pratiquer en laboratoire, car c'est le pH de l'eau purifiée. La mesure se fait donc simplement par dissolution dans de l'eau purifiée.

Selon l'invention, la composition selon l'invention est apte à libérer (ou dissoudre) le citrate de potassium *in vitro* dans un milieu de dissolution de solution tamponnée à pH 1,3 réalisé avec un appareil de dissolution de type 2, conforme à la Pharmacopée Européenne (Ph. Eur.) 2.9.3 « *Essai de dissolution des formes solides* », à un taux de 2 à 15% en 15 minutes, de 15 à 25% en 30 minutes, et de 30 à 50% en une heure.

Ce pH de 1,3 est représentatif du milieu acide de l'estomac.

De façon générale, la dissolution *in vitro* de la composition selon l'invention dans un milieu de dissolution donné, selon les conditions décrites plus haut, se produit à pH indépendant. Cela veut dire que, quelque soit le pH du milieu de dissolution dans une plage entre 1,3 et 7, la dissolution se produit selon la même cinétique. La demanderesse a choisi ici deux milieux de dissolution différents, chacun caractérisé par son pH, à savoir pH 1,3 et pH 7, pour définir ce profil de façon caractéristique, selon un test facilement reproductible *in vitro.*

Pour ces mesures, un gramme de composition pharmaceutique, ce qui correspondant à une unité de dosage, est placée dans un banc de dissolution de type Pharmatest modèle PTW S3C, dans lequel les conditions de températures sont 37°C ± 0,5°C, et la vitesse de rotation est de 100rpm (tours par minute). Le volume du bol de dissolution est de 1L et le milieu de dissolution utilisé est de l'eau purifiée à pH 7 ou une solution tamponnée à pH 1,3.

Le citrate de potassium est dosé comme il est connu de l'homme du métier. Par exemple, le citrate de potassium libéré est dosé avec un photomètre de flamme, la technique analytique ayant été validée selon les recommandations ICH CPMP/ICH/381/95 - ICH Q2 (R1).

Ainsi, la libération d'une quantité trop importante de citrate de potassium est évitée dès que la forme entre en contact avec le liquide de dissolution avantageusement selon l'invention. Cela permet d'éviter un effet de matraquage de l'organisme du patient, contrairement aux compositions de l'art antérieur. En outre, la cinétique de dissolution du sel précurseur du cycle de Krebs est avantageusement ralentie en début de dissolution, ce qui améliore notablement la tolérance gastrique du produit par rapport aux produits de l'art antérieur.

De préférence, le citrate de potassium est totalement dissous (taux de dissolution de 100%) en environ quatre heures, que le pH soit de 7 ou de 1,3.

La composition selon l'invention comprend de 40% à 80%, de préférence de 50 à 70%, en poids de citrate de potassium sur la base du poids total de la composition. Le citrate de potassium est ainsi présent à dose physiologiquement efficace ou représentant un multiple ou un sous-multiple d'une dose efficace pour un patient type.

Ceci représente un taux de principe actif, en poids par rapport au poids total de la composition, important par rapport à ce qui est connu. Cela permet avantageusement de minimiser le volume de la composition pharmaceutique, et donc le volume de prise journalière. Par voie de conséquence, on obtient ainsi une meilleure acceptation par le patient.

Ceci est particulièrement appréciable pour les prises de composition en dose élevée et/ou pour les traitements thérapeutiques pédiatriques.

L'agent d'enrobage est généralement choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane et l'oxyde de polyéthylène, les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne. L'agent d'enrobage est de façon préférée selon l'invention un polymère d'éthylcellulose.

Selon un mode de réalisation de l'invention, l'enrobage comporte, outre un agent d'enrobage tel que choisi dans la liste ci-dessus, un agent aromatisant et/ou un colorant.

L'épaisseur et l'homogénéité de l'enrobage est un paramètre important de l'invention, car il influence la diffusion du précurseur du cycle de Krebs au travers de l'enrobage et donc la cinétique de dissolution de ce précurseur. Le choix de la nature et de la quantité de l'agent d'enrobage utilisé est un paramètre important de l'invention.

La composition pharmaceutique selon l'invention comprend généralement de 0,01% à 5%, de préférence de 0,01% à 2% en poids, de façon encore plus préférée de 1,4 à 2,5%, d'agent d'enrobage par rapport au poids total de la composition.

La composition pharmaceutique selon l'invention peut comprendre en outre :
- de 10% à 40%, de préférence de 25% à 35% en poids, par rapport au poids total de la composition, d'un liant choisi parmi les celluloses microcristallines, la polyvidone, la polyvinylpyrrolidone, la copovidone, la gomme laque (shellac), la gélatine, les polyméthacrylates, les résines synthétiques, les acrylates, la maltodextrine, et les amidons, et de préférence le liant comporte au moins une cellulose microcristalline ;
- de 0,01% à 5%, de préférence de 0,02% à 3% en poids, par rapport au poids total de la composition, d'un agent d'écoulement (ou lubrifiant) choisi parmi l'acide stéarique, le polyéthylène glycol, le stéarate de magnésium, le stéarate de calcium, le stéarate de zinc, le talc, le dioxyde de silice, l'huile de castor hydrogénée, le glycéryl béhénate, et le glycéryl palmitostéarate, et de préférence l'agent d'écoulement est choisi parmi le stéarate de magnésium et le glycéryl béhénate ; et/ou
- tout excipient pharmaceutique approprié, en une quantité classiquement utilisée dans le domaine considéré, par exemple de 0,0001% à 20% du poids total de la composition.

L'excipient pharmaceutique est généralement inerte, c'est-à-dire inactif et non toxique, et acceptable d'un point de vue pharmaceutique. Un tel excipient est le plus souvent choisi parmi les diluants, liants, désagrégeants, agents d'écoulement, lubrifiants, colorants autorisés par l'Administration compétente, dispersants, solubilisants, stabilisants, conservateurs, plastifiants et agents aromatisants. Un tel excipient peut être aussi un support par exemple choisi dans le groupe formé par les celluloses telles que l'hydroxyméthylcellulose, la carboxyméthylcellulose, les cyclodextrines, le polysorbate 80, le mannitol, la gélatine, le lactose, les huiles végétales, les huiles animales, les carbonates, les amidons et l'acacia.

De préférence, tous les micro-comprimés ont la même composition et présentent un taux de dissolution similaire, qui est le taux de dissolution pouvant caractériser la composition pharmaceutique de l'invention.

En outre, le coeur du micro-comprimé de la composition selon l'invention peut comprendre au moins une matrice à libération prolongée, de préférence à une teneur comprise dans une fourchette de 10% à 30%, de façon encore plus préférée de 15% à 25%, en poids par rapport au poids total de la composition. Une telle matrice à libération prolongée est de préférence choisie parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane et l'oxyde de polyéthylène, les cires de type cire de paraffine, cire d'abeille ou de Carnauba, les copolymères d'ammonium méthacrylate de type A et B tels que décrits dans la Pharmacopée Européenne, et les polyacrylates de dispersion environ 30% tels que décrits dans la Pharmacopée Européenne.

Une telle matrice à libération prolongée se définit de la façon suivante.

La Pharmacopée Européenne (Ph. Eur.) définit, parmi les comprimés à libération modifiée, les comprimés à libération prolongée, les comprimés à libération retardée et les comprimés à libération séquentielle. Les comprimés à libération modifiée sont des comprimés, enrobés ou non, qui sont préparés avec des excipients spéciaux, ou par des procédés particuliers, ou les deux, visant à modifier, la vitesse, le lieu ou le moment de libération de la ou des substances actives.

En général, les comprimés à libération prolongée sont les comprimés permettant de libérer une substance active de façon prolongée dans le temps et selon une cinétique déterminée. Cela est de préférence effectué en réalisant un coeur de comprimé, ou un comprimé nu (i.e. sans enrobage) utilisant une matrice à libération prolongée dans laquelle est présente la ou les substances actives. Une matrice à libération prolongée est en général un système matriciel, le plus souvent un réseau polymérique, qu'il soit hydrophile ou lipophile. La diffusion de la ou les substances actives au sein de ce réseau est généralement influencée non seulement par les propriétés physico-chimiques inhérentes à cette ou ces substances actives (telle que solubilité, poids moléculaire ...), mais également par celles caractérisant le réseau matriciel (tels que: hydrophilie, degré de polymérisation, vitesse de gélification, érosion).

Selon le mode de réalisation de l'invention, la composition pharmaceutique se présente sous la forme de micro-comprimés. ayant une taille comprise dans une fourchette de 2 à 4 mm La Pharmacopée Européenne (Ph. Eur.) définit un comprimé comme une préparation solide contenant une unité de prise d'une ou plusieurs substances actives. Les comprimés sont obtenus en agglomérant par compression un volume constant de particules, ou par un autre procédé de fabrication approprié tel que l'extrusion, le moulage ou la cryodessication (lyophilisation). Les comprimés sont destinés à la voie orale. Les comprimés se présentent généralement sous la forme d'un cylindre droit dont les faces inférieures et supérieures peuvent être plates ou convexes et les bords biseautés. La taille d'un comprimé, ou dimension moyenne, est donc généralement le diamètre de ce cylindre, ou un équivalent. Par contre si la hauteur du cylindre est importante, et supérieure au diamètre du cylindre, la taille du comprimé est la hauteur de ce cylindre.

Par "micro-comprimé", on entend selon l'invention un comprimé de taille comprise dans une fourchette de 2 à 4 mm (en général avec une précision sur la taille de ±10%). Les micro-comprimés ont la même composition et présentent chacun un taux de dissolution similaire, qui est le taux de dissolution pouvant caractériser la composition pharmaceutique de l'invention. Ce taux de dissolution est couramment établi sur la base d'une unité de la préparation, soit dans le cadre de l'invention d'un gramme de micro-comprimés. Du fait de la petite taille du micro-comprimé, un seul micro-comprimé ne sera pas suffisant pour une prise, et à chaque prise, plusieurs micro-comprimés seront administrés.

Un avantage de la forme en micro-comprimés est que la prise par le patient est facilitée, par rapport à une prise d'un seul comprimé de volume plus important. Ceci est particulièrement avantageux lorsque le patient est un enfant.

On comprend que selon l'invention, le patient pourra ingérer plusieurs micro-comprimés à chaque prise, selon la dose thérapeutique qui lui est appropriée (dose journalière divisée par le nombre de prises par jour). Ainsi une prise de médicament correspond à plusieurs micro-comprimés, c'est-à-dire à un ensemble de micro-comprimés. L'invention vise donc à couvrir également un ensemble de micro-comprimés, correspondant à une prise thérapeutique. L'homme du métier est à même d'évaluer le nombre de micro-comprimés correspondant à une dose thérapeutique, en fonction des besoins de la personne, de son âge, de son poids, en fonction de la quantité de sel de citrate par micro-comprimé, ainsi que le nombre de prises par jour.

Les micro-comprimés selon l'invention sont enrobés, ce qui permet un masquage du goût.

Les micro-comprimés selon l'invention sont particulièrement bien adaptés au traitement des lithiases urinaires se manifestant à un pH physiologique et/ou lors d'acidose urinaire et/ou lors d'hypocitraturie et/ou lors d'hypercalciurie et/ou lors d'hyperoxalurie, du fait de leur profil de libération optimale.

La composition selon la présente invention peut être utilisée chez le mammifère, plus précisément chez l'homme, et tout particulièrement chez l'enfant.

Le procédé de fabrication de la composition pharmaceutique selon l'invention comprend généralement les trois étapes successives décrites ci-après.

La première étape est une étape de mélange du citrate de potassium de préférence du seul principe actif, avec les autres ingrédients constituant le coeur de la composition pharmaceutique selon l'invention. Le mélange est par exemple réalisé dans un mélangeur par gravité de type Stuart STR4, mais peut être réalisé dans tout autre type de mélangeur industriel.

La deuxième étape est une étape de fabrication des comprimés, à partir du mélange issu de la première étape, généralement réalisée par une première opération de compression directe dans une presse rotative, par exemple pour la fabrication de micro-comprimés de dimension 2 mm (de type PR12) à l'aide de six supports possédant chacun une tête à six poinçons de 2mm. Cette deuxième étape comprend ensuite une seconde opération de dépoussiérage des comprimés fabriqués au cours de la première opération.

La troisième étape est une étape d'enrobage, par l'agent d'enrobage, des micro-comprimés issus de la deuxième étape. L'agent d'enrobage est généralement appliqué sous forme de solution ou de suspension dans des conditions qui favorisent l'évaporation du solvant.

Selon un mode de réalisation de l'invention, la composition comprend de 55% à 70% de citrate de potassium, de 20 à 40% de cellulose microcristalline, de 0,02% à 3% de stéarate de magnésium, de 0,01% à 1% de glycéryl béhénate et de 1 à 3% de polymère d'éthyl cellulose, par rapport au poids total de la composition.

L'invention a aussi pour objet une composition selon l'invention pour son utilisation comme médicament.

L'invention a également pour objet une composition selon l'invention pour son utilisation comme médicament dans le traitement et/ou la prévention des lithiases urinaires se manifestant à un pH physiologique et/ou lors d'acidose urinaire et/ou lors d'hypocitraturie et/ou lors d'hypercalciurie et/ou lors d'hyperoxalurie.

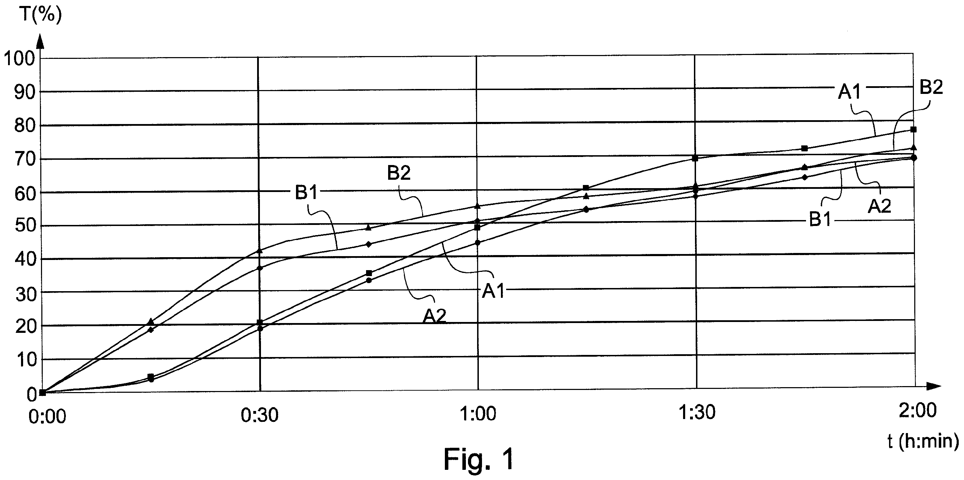

L'invention est illustrée par la figure 1 ci-jointe, qui représente le profil de dissolution en taux T de dissolution (pourcentage de principe actif -citrate de potassium) en fonction de la durée t (min) pour quatre compositions / conditions différentes identifiées par A1 et A2 (composition selon l'invention et pH respectifs de 7 et 1,3) et B1 et B2 (compositions selon l'art antérieur, Urocit-K, et pH respectifs de 7 et 1,3).

La figure 1 est commentée dans l'exemple ci-après, qui illustre l'invention sans pour autant la limiter.

### Exemple :

Un lot de micro-comprimés de taille (diamètre moyen) 2mm est réalisé selon le procédé décrit précédemment à savoir, une étape de mélange des poudres, suivie d'une étape de compression, puis d'une étape d'enrobage. Ce lot est le lot A, constitué de 200g de micro-comprimés. Ces micro-comprimés ont la composition suivante :
Citrate de potassium (principe actif, source Dr Paul Lohmann) : 66,9%
Cellulose microcristalline (liant, Ceolus® KG-802 de la société Asahi) : 19,7%
Cellulose microcristalline (liant, Ceolus® UF-711 de la société Asahi) : 9,8%
Stéarate de magnésium (agent d'écoulement) : 2,0%
Glyceryl béhénate (agent lubrifiant, référence commerciale Compritol® ATO 888 de la société GATTEFOSSE) : 0,01 % ;
Polymère d'éthyl cellulose (agent d'enrobage, référence commerciale Ethocel® 20 standard premium de la société Dow) : 1,66%.

Ces micro-comprimés sont très bien acceptés et tolérés par les patients. En outre, ils sont sans goût et faciles à avaler.

La figure 1 montre le profil de dissolution in vitro d'un gramme de ces micro-comprimés dans l'eau, dans les conditions décrites ci-après, sur une durée de 2 heures. Les micro-comprimés A ont été placés dans un banc de dissolution Pharmatest modèle PTW S3C, dans lesquelles les conditions de températures sont 37°C ± 0,5°C, et la vitesse de rotation est de 100rpm. Selon le milieu de dissolution, on a obtenu deux courbes différentes respectivement A1 et A2 pour une solution d"eau purifiée à pH 7 et pour une solution tamponnée à pH 1,3. Les courbes A1 et A2 sont sensiblement identiques, surtout pour une durée inférieure à 1 h voire trente minutes.

Comme illustré sur la Figure 1, les micro-comprimés du lot A sont aptes à libérer le sel de citrate *in vitro* dans un milieu de dissolution d'eau purifiée à pH 7, ainsi que dans une solution tamponnée à pH 1,3, à un taux respectivement de 4,5 et 3,7% en 15 minutes, de 20,6 et 18,6% en 30 minutes, et de 48,6 et 44,0% en une heure

De comprimés génériques d'Urocit-K ont été achetés dans le commerce sous la forme d'une boîte de 90 comprimés de référence « Potassium citrate (1080mg) Tabs MFG RISING - Generic for Urocit-k 1080mg (10 meq) Tablets RX 1505494-03363 », vendue par WALGREENS, Indianapolis, USA. L'expérience a été répétée sur ces comprimés. On a obtenu deux courbes différentes respectivement B1 et B2 pour une solution d"eau purifiée à pH 7 et pour une solution tamponnée à pH 1,3. Les courbes B1 et B2 sont sensiblement identiques, surtout pour une durée inférieure à 1 h voire trente minutes. Les courbes B1 et B2 croisent les courbes A1 et A2 pour une durée comprise entre 1h et 1h30.

Comme illustré sur la figure 1, les comprimés d'Urocit-K A sont aptes à libérer le sel de citrate *in vitro* dans un milieu de dissolution d'eau purifiée à pH 7, ainsi que dans une solution tamponnée à pH 1,3, à un taux respectivement de 37,0 et 42,1% en 30 minutes, et de 50,5 et 55,1% en une heure.

La figure 1 montre que les courbes de dissolution selon l'invention (A1 et A2) et selon l'art antérieur (B1 et B2) sont très différentes, en particulier au démarrage de la dissolution, auquel les valeurs des pentes sont très distinctes. Cela démontre une libération progressive et beaucoup plus lente du principe actif qu'est le sel de citrate selon l'invention, évitant un effet de matraquage de l'organisme qui serait susceptible de créer une alcalose. En outre, cette différence permet certainement d'expliquer en partie l'amélioration importante de la tolérance gastrique par rapport à l'Urocit-K.

## Revendications

1. Composition pharmaceutique solide à usage oral sous forme d'au moins un comprimé,
ledit comprimé étant constitué d'un coeur comprenant du citrate de potassium en tant que principe actif, et d'un enrobage comprenant au moins un agent d'enrobage, ladite composition comprenant de 40% à 80%, de préférence de 50% à 70%, en poids de citrate de potassium sur la base du poids total de la composition,
ladite composition se présentant sous la forme de micro-comprimés, ayant de préférence une taille comprise dans une fourchette de 2 à 4 mm,
ladite composition étant apte à libérer le citrate de potassium *in vitro,* aussi bien dans un milieu de dissolution d'eau purifiée à pH 7 que dans un milieu de dissolution de solution tamponnée à pH 1,3 avec un appareil de dissolution de type 2, conforme à la Pharmacopée Européenne 2.9.3. « *Essai de dissolution des formes solides* », à un taux de 2 à 15% en 15 minutes, de 15 à 25% en 30 minutes, et de 30 à 50% en une heure,
pour son utilisation comme médicament dans le traitement et/ou la prévention des lithiases urinaires se manifestant à un pH physiologique et/ou lors d'acidose urinaire et/ou lors d'hypocitraturie et/ou lors d'hypercalciurie et/ou lors d'hyperoxalurie.

2. Composition selon la revendication 1, telle qu'elle comprend de 0,01% à 5%, de préférence de 0.01% à 2% en poids, de façon encore plus préférée de 1,4 à 2,5% d'agent d'enrobage par rapport au poids total de la composition.

3. Composition selon l'une des revendications 1 ou 2, telle que l'agent d'enrobage est choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane, l'oxyde de polyéthylène, les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne, et l'agent d'enrobage est de préférence un polymère d'éthylcellulose.

4. Composition selon l'une quelconque des revendications 1 à 3, telle qu'elle comprend en outre de 10% à 40%, de préférence de 25 à 35%, en poids, par rapport au poids total de la composition, d'un liant choisi parmi les celluloses microcristallines, la polyvidone, la polyvinylpyrrolidone, la copovidone, la gomme laque, la gélatine, les polyméthacrylates, les résines synthétiques, les acrylates, la maltodextrine, et les amidons, et de préférence le liant comporte au moins une cellulose microcristalline.

5. Composition selon l'une quelconque des revendications 1 à 4, telle qu'elle comprend en outre de 0,01% à 5%, de préférence de 0,02% à 3% en poids, par rapport au poids total de la composition, d'un agent d'écoulement choisi parmi l'acide stéarique, le polyéthylène glycol, le stéarate de magnésium, le stéarate de calcium, le stéarate de zinc, le talc, le dioxyde de silice, l'huile de castor hydrogénée, le glycéryl béhénate, et le glycéryl palmitostéarate, et de préférence l'agent d'écoulement est choisi parmi le stéarate de magnésium et le glycéryl béhénate.

6. Composition selon l'une quelconque des revendications 1 à 5, telle que le coeur du comprimé comprend au moins une matrice à libération prolongée, de préférence à une teneur comprise dans une fourchette de 10% à 30%, de préférence de 25 à 35%, en poids par rapport au poids total de la composition.

7. Composition selon l'une quelconque des revendications 1 à 6, telle qu'elle comprend de 55% à 70% de citrate de potassium, de 20 à 40% de cellulose microcristalline, de 0,02% à 3% de stéarate de magnésium, de 0,01% à 1% de glycéryl béhénate et de 1 à 3% de polymère d'éthyl cellulose, par rapport au poids total de la composition.

## Patentansprüche

1. Feste pharmazeutische Zusammensetzung zur oralen Verwendung in Form mindestens einer Tablette,
wobei die Tablette aus einem Kern mit Kaliumcitrat als aktiven Wirkstoff und einer Beschichtung mit mindestens einem Beschichtungsmittel besteht, wobei die Zusammensetzung auf der Basis des Gesamtgewichts der Zusammensetzung 40 Gew.-% bis 80 Gew.-%, vorzugsweise 50 Gew.-% bis 70 Gew.-% Kaliumcitrat umfasst,
wobei die Zusammensetzung in Form von Mikrotabletten vorliegt, die vorzugsweise eine Größe in einem Bereich von 2 bis 4 mm haben,
wobei die Zusammensetzung das Kaliumcitrat sowohl in einem Lösungsmedium aus gereinigtem Wasser mit einem pH-Wert von 7 als auch in einem Lösungsmedium aus einer gepufferten Lösung mit einem pH-Wert von 1,3 mittels einer Lösungsvorrichtung vom Typ 2 gemäß der Europäischen Pharmakopöe 2.9.3. "*Test zur Auflösung fester Formen*" mit einer Rate von 2 bis 15% innerhalb von 15 Minuten, von 15 bis 25% innerhalb von 30 Minuten und von 30 bis 50 % innerhalb einer Stunde *in vitro* freisetzen kann,
für ihre Verwendung als Arzneimittel bei der Behandlung und/oder der Vorbeugung von Urolithiasen, die bei einem physiologischen pH-Wert und/oder bei einer Azidose der Harnwege und/oder bei einer Hypocitraturie und/oder bei einer Hypercalciurie und/oder bei einer Hyperoxalurie auftreten.

2. Zusammensetzung nach Anspruch 1, wobei sie bezogen auf das Gesamtgewicht der Zusammensetzung 0,01 Gew.-% bis 5 Gew.-%, vorzugsweise 0,01 Gew.-% bis 2 Gew.-% und in einer weiter bevorzugteren Weise 1,4 Gew.-% bis 2,5 Gew.-% eines Beschichtungsmittels umfasst.

3. Zusammensetzung nach einem der Ansprüche 1 oder 2, wobei das Beschichtungsmittel aus den Alginaten, den Carboxyvinylpolymeren, den Salzen aus Natrium-Carboxymethylcellulose, den Cellulosederivaten einschließlich der Polymere Hydroxypropylmethylcellulose, Hydroxypropylethylcellulose, Hydroxypropylcellulose, Hydroxyethylcellulose, Methylcellulose, Ethylcellulose, Xanthangummi, Polyethlylenoxid, den Wachsen vom Typ Paraffinwachs, Bienenwachs oder Carnaubawachs, den Ammoniummethacrylat-Copolymeren vom Typ A und B, wie in der Europäischen Pharmakopöe beschrieben, und den Polyacrylat-Dispersionen etwa 30%, wie in der Europäischen Pharmakopöe beschrieben, ausgewählt ist und es sich bei dem Beschichtungsmittel vorzugsweise um ein Ethylcellulosepolymer handelt.

4. Zusammensetzung nach einem der Ansprüche 1 bis 3, wobei sie bezogen auf das Gesamtgewicht der Zusammensetzung ferner 10 Gew.-% bis 40 Gew.-%, vorzugsweise 25 Gew.-% bis 35 Gew.-% eines Bindemittels umfasst, das aus den mikrokristallinen Cellulosen, Polyvidon, Polyvinylpyrrolidon, Copovidon, Schellack, Gelatine, den Polymethacrylaten, den Kunstharzen, den Acrylaten, Maltodextrin und den Stärken ausgewählt ist und das Bindemittel vorzugsweise zumindest eine mikrokristalline Cellulose umfasst.

5. Zusammensetzung nach einem der Ansprüche 1 bis 4, wobei sie bezogen auf das Gesamtgewicht der Zusammensetzung ferner 0,01 Gew.-% bis 5 Gew.-%, vorzugsweise 0,02 Gew.-% bis 3 Gew.-% eines Fließmittels umfasst, das aus Stearinsäure, Polyethylenglycol, Magnesiumstearat, Calciumstearat, Zinkstearat, Talk, Siliciumdioxid, hydriertem Rizinusöl, Glycerylbehenat und Glycerylpalmitostearat ausgewählt ist und das Fließmittel vorzugsweise aus Magnesiumstearat und Glycerylbehenat ausgewählt ist.

6. Zusammensetzung nach einem der Ansprüche 1 bis 5, wobei der Kern der Tablette mindestens eine Retardmatrix umfasst, vorzugsweise in einem Gehalt in einem Bereich von 10 Gew.-% bis 30 Gew.-%, vorzugsweise von 25 Gew.-% bis 35 Gew.-% bezogen auf das Gesamtgewicht der Zusammensetzung.

7. Zusammensetzung nach einem der Ansprüche 1 bis 6, wobei sie bezogen auf das Gesamtgewicht der Zusammensetzung 55 % bis 70 % Kaliumcitrat, 20 % bis 40 % mikrokristalline Cellulose, 0,02 % bis 3 % Magnesiumstearat, 0,01 % bis 1 % Glycerylbehenat und 1 % bis 3 % Ethylcellulosepolymer umfasst.

## Claims

1. Solid pharmaceutical composition for oral use in the form of at least one tablet,
said tablet consisting of a core comprising potassium citrate as active ingredient, and a coating comprising at least one coating agent, said composition comprising from 40% to 80%, preferably from 50 to 70%, by weight of potassium citrate salt based on the total weight of the composition,
the composition being in the form of microtablets, preferably having a size comprised within the range of from 2 to 4 mm,
said composition being able to release the potassium citrate salt *in vitro,* both in a dissolution medium of purified water at pH 7 and in a dissolution medium of solution buffered at pH 1.3, with a dissolution apparatus of type 2, according to the European Pharmacopoeia 2.9.3 "*Dissolution test for solid dosage forms*", at a rate of from 2 to 15% in 15 minutes, from 15 to 25% in 30 minutes, and from 30 to 50% in one hour,
for use as a medicament in the treatment and/or prevention of urinary lithiases occurring at a physiological pH and/or during urinary acidosis and/or during hypocitraturia and/or during hypercalciuria and/or during hyperoxaluria.

2. Composition according to claim 1, such that it comprises from 0.01% to 5%, preferably from 0.01% to 2% by weight, even more preferably from 1.4 to 2.5%, of coating agent relative to the total weight of the composition.

3. Composition according to one of claims 1 or 2, such that the coating agent is selected from alginates, carboxyvinyl polymers, sodium salts of carboxymethyl cellulose, cellulose derivatives including the polymers hydroxypropyl methylcellulose, hydroxypropyl ethylcellulose, hydroxypropyl cellulose, hydroxyethyl cellulose, methylcellulose, ethylcellulose, xanthan gum, polyethylene oxide, waxes such as paraffin wax, beeswax or carnauba wax, ammonium methacrylate copolymers of type A and B as described in the European Pharmacopoeia, and polyacrylates of about 30% dispersion as described in the European Pharmacopoeia, and the coating agent is preferably an ethylcellulose polymer.

4. Composition according to any one of claims 1 to 3, such that it further comprises from 10% to 40%, preferably from 25 to 35%, by weight, relative to the total weight of the composition, of a binder selected from microcrystalline celluloses, polyvidone, polyvinylpyrrolidone, copovidone, shellac, gelatin, polymethacrylates, synthetic resins, acrylates, maltodextrin, and starches, and preferably the binder comprises at least one microcrystalline cellulose.

5. Composition according to any one of claims 1 to 4, such that it further comprises from 0.01% to 5%, preferably from 0.02% to 3% by weight, relative to the total weight of the composition, of a flow agent selected from stearic acid, polyethylene glycol, magnesium stearate, calcium stearate, zinc stearate, talc, silica, hydrogenated castor oil, glyceryl behenate, and glyceryl palmitostearate, and preferably the flow agent is selected from magnesium stearate and glyceryl behenate.

6. Composition according to any one of claims 1 to 5, such that the tablet core comprises at least one sustained-release matrix, preferably with a content comprised within a range of from 10% to 30%, preferably from 15% to 25%, by weight relative to the total weight of the composition.

7. Composition according to any one of claims 1 to 6, such that it comprises from 55% to 70% potassium citrate, from 20 to 40% microcrystalline cellulose, from 0.02% to 3% magnesium stearate, from 0.01% to 1% glyceryl behenate and from 1 to 3% ethylcellulose polymer, relative to the total weight of the composition.
